# EUROPEAN PATENT SPECIFICATION

(11) **EP 2 097 022 B1**
(45) Date of publication and mention of the grant of the patent: **23.07.2014**
(21) Application number: 07853141.5
(22) Date of filing: 20.11.2007
(51) Int. Cl.: A61B 17/34, A61B 19/00, A61B 17/00

(54) **MRI DETECTABLE OBTURATOR**
MRT DETEKTIERBARER OBTURATOR
OBTURATEUR POUVANT ÊTRE DÉTECTÉ PAR IRM

(30) Priority: 24.11.2006 US 860887 P; 30.11.2006 US 872020 P; 30.10.2007 US 980302; 19.11.2007 US 985997
(43) Date of publication of application: 09.09.2009
(73) Proprietor: Senorx, Inc., Tempe, AZ 85280-1740 (US)
(72) Inventor: LUBOCK, Paul, Laguna Niguel, CA 92677 (US); JONES, Michael L., San Clemente, CA 92673 (US); BROADAWAY, Ethan, Huntington Beach, CA 92647 (US); LOUW, Frank R., Carlsbad, CA 92009 (US)
(74) Representative: Tomlinson, Edward James
(86) International application number: PCT/US2007/024255
(87) International publication number: WO 2008/063638

(56) References cited:
- WO-A-03/105940
- WO-A-2005/013832
- WO-A-2008/016551
- US-A- 4 989 608
- US-A1- 2006 036 165
- US-A1- 2006 241 385
- US-B1- 6 628 982

## Description

### FIELD OF THE INVENTION

This invention relates generally to the fields of medical treatment devices and methods. In particular, the invention relates to devices for marking a body site, such as a site from which cancerous, pre-cancerous, or other tissue has been or will be removed.

### BACKGROUND OF THE INVENTION

In diagnosing and treating certain medical conditions, it is often desirable to perform a biopsy, in which a specimen or sample of tissue is removed for pathological examination, tests and analysis. A biopsy typically results in a biopsy cavity occupying the space formerly occupied by the tissue that was removed. As is known, obtaining a tissue sample by biopsy and the subsequent examination are typically employed in the diagnosis of cancers and other malignant tumors, or to confirm that a suspected lesion or tumor is not malignant. Treatment of cancers identified by biopsy may include subsequent removal of tissue surrounding the biopsy site, leaving an enlarged cavity in the patient's body. Cancerous tissue is often treated by application of radiation, by chemotherapy, or by thermal treatment (e.g., local heating, cryogenic therapy, and other treatments to heat, cool, or freeze tissue).

Cancer treatment may be directed to a natural cavity, or to a cavity in a patient's body from which tissue has been removed, typically following removal of cancerous tissue during a biopsy or surgical procedure. For example, U.S. Pat. No. 6,923,754 to Lubock and U.S. Pat. Application Serial No. 10/849,410 to Lubock describe devices for implantation into a cavity resulting from the removal of cancerous tissue which can be used to deliver cancer treatments to surrounding tissue. One form of radiation treatment used to treat cancer near a body cavity remaining following removal of tissue is "brachytherapy" in which a source of radiation is placed near to the site to be treated.

Lubock above describes implantable devices for treating tissue surrounding a cavity left by surgical removal of cancerous or other tissue that includes an inflatable balloon constructed for placement in the cavity. Such devices may be used to apply one or more of radiation therapy, chemotherapy, and thermal therapy to the tissue surrounding the cavity from which the tissue was removed. The delivery lumen of the device may receive a solid or a liquid radiation source. Radiation treatment is applied to tissue adjacent the balloon of the device by placing radioactive material such as radioactive "seeds" in a delivery lumen. Such treatments may be repeated if desired.

A radiation source such as a miniature or micro-miniature x-ray tube may also be used (e.g. U.S. Patent No. 6,319,188). The x-ray tubes are small, flexible and are believed to be maneuverable enough to reach the desired treatment location within a patient's body. The radiation source is to be removed following each treatment session, or remains in place as long as the balloon remains within the body cavity. Other inflatable treatment delivery devices and systems may be used to treat cancer in tissue adjacent a body cavity.

When performing an image guided biopsy procedure an obturator is used as a place holder and is located in tissue so that its distal tip will be located at the point in the patient's body where the biopsy is to be taken or where a biopsy site marker or tissue marker is to be placed after a biopsy procedure. Subsequent images are acquired that can confirm the correct placement of the obturator. When the obturator is placed at the desired location within the body, blood can enter the lumen of the obturator prior to delivery of the tissue markers. This backflow of blood into the obturator creates a risk of blood clotting.

Current obturators are constructed of homogeneous materials. During magnetic resonance imaging (MRI) guided biopsies, the tip of the obturator is located by indexing through many cross sectional views (typically every 2 mm, but higher and lower discriminations are possible). The material of the obturator will be distinguishable in the cross sectional images to a varying degree depending on the morphology of the tissue and the obturator's own material makeup. Since the prior obturators were homogeneous, the signature of the obturators will not vary from one cross-sectional image to the next along its length. The tip of the obturator is located by selecting the first cross-sectional image in which the obturator is not seen. This result can be visually ambiguous depending on the relative strength of the image signature of the obturator compared to the surrounding tissue.

US 4,989,608 describes a catheter to be imaged using MRI incorporating ferromagnetic material at desired portions of a flexible member or liquid or gel contrast agent within a lumen of the catheter. US 2006/0241385 A1 describes an automated biopsy device which may be used introduce a marker fluid filled obturator into a patient. US 6,628,982 describes an internal marker device comprising a flexible lumen containing MRI imaging material. WO 2005/013832 A1 describes a medical closure device including membranes through which a needle may be inserted. US 2006/0036165 A1 describes a system for introducing tissues site markers into a patient comprising a cannula containing the markers and a piston for deploying the markers through an opening in the tip of the cannula.

WO2003/105940 describes a plugged tip delivery tube for marker placement.

### SUMMARY OF THE INVENTION

This invention is generally directed to marking a patient's body cavity or other intracorporeal site (hereinafter collectively referred to as a body cavity) and devices for such marking. The invention is particularly suitable for marking an intracorporeal site of a lesion by magnetic resonance imaging (MRI).

More specifically, the invention is directed to an obturator with an elongated shaft having a distal shaft portion which is detectable by MRI but which does not significantly interfere with imaging of an adjacent tissue, particularly an adjacent lesion. Preferably, the distal shaft portion of the obturator contains or has incorporated therein an MRI detectable agent in amounts effective to provide a readily apparent T1 MRI image within the outline of the distal shaft portion of the obturator.

The obturator having features of the invention operates as a place-holder during an MRI guided procedure such as a biopsy. The distal end of the obturator is placed where the procedure is to be performed or one or more intracorporeal objects or bodies are to be delivered.

In one embodiment having features of the present invention the device includes an obturator which has an elongated shaft with a internal lumen, a proximal end, and a substantially sealed distal end which prevents or minimizes the backflow of body fluids, such as blood, though the lumen of the obturator. The substantially sealed distal end can be a penetratable membrane or may have petals or a duckbill-type valve which are configured to allow passage of one or more intracorporeal objects or a delivery tube with one or more intracorporeal objects therethrough while preventing or minimizing entry of body fluids into the inner lumen of the obturator. Preferably, the obturator is configured to fit within a procedure cannula, e.g. a cannula of a biopsy device, for example, the cannula of SenoRx's EnCor™ Magnetic Resonance Imaging Breast Biopsy System. The cannula provides access to the desired location within the patient's body.

The delivery tube has a delivery lumen configured to contain one or more intracorporeal objects. The distal tip is configured to penetrate the substantially closed distal end of the obturator so that the intracorporeal bodies can be delivered while the obturator is in place within the body. The shape of the distal tip may be sharp or needle like when the distal end of the obturator has a pierceable membrane or it may be blunt or rounded when the distal end of the obturator is petalled or has a one-way valve.

The delivery tube preferably further includes a plunger having an elongated shaft with a proximal portion and a distal portion. The plunger is configured to be slidably disposed within the lumen of the delivery tube and is located proximal to the one or more intracorporeal objects within the lumen thereof. When the plunger is extended distally within the lumen, the distal end thereof moves one or more intracorporeal objects toward and eventually through the distal end of the delivery tube. The plunger preferably has an enlarged proximal end to prevent the distal portion of the plunger from advancing too far within the delivery lumen. Alternatively, a fluid may be used to advance the intracorporeal objects through the opening in the distal end of the delivery tube.

In use, the obturator is placed at a desired location within a patient's body. The delivery tube is advanced distally within the obturator until the distal tip passes through the substantially closed distal end of the obturator. Next, the plunger is advanced distally within the delivery tube so that at least one intracorporeal object is pushed though the opening of the distal tip of the delivery tube.

The MRI detectable distal shaft portion of the obturator includes an MRI detectable mass which is capable of producing a significant image signature at the location in the patient's body where the distal portion of the obturator is placed without interfering with the imaging of adjacent tissue. In one embodiment the distal shaft portion has an MRI detectable material incorporated into a preferably solid distal shaft portion. In another embodiment the distal shaft portion has an inner portion or lumen filled with an MRI detectable mass. For example, an inner lumen extending within the distal shaft portion is filled with a gel having an MRI detectable material incorporated therein or filled with a solid MRI detectable element. The distal shaft portion has an effective amount of MRI detectable material so as to provide a clear, T1-weighted image within an outline of the distal shaft portion upon magnetic resonance imaging thereof.

The obturator embodying features of the invention may be used with a trocar assembly which has a trocar with a tissue penetrating distal tip and an introducer or trocar sheath which remains at the site after the trocar is removed. The introducer or trocar sheath may be similar to the split sheath trocar introducer which is described in co-pending provisional application Serial No. 60/964,079. As described therein the split sheath may have a short cylindrical distal portion that is configured to provide a friction fit with the trocar proximal to the tissue penetrating distal tip. Preferably, the slit in the introducer sheath extends proximally from the cylindrical distal portion and widens in the proximal direction. The short cylindrical distal portion of the sheath preferably has a weakened or scored portion in alignment with the slit so that when the trocar is removed and an another instrument having a transverse dimension or expandable to a transverse dimension slightly larger than the internal transverse dimension of the short cylindrical sheath portion, the cylindrical section will tear or break to facilitate the removal of the sheath without removal of the instrument.

These and other advantages of the present invention are described in more detail in the following written description and the accompanying exemplary drawings.

### BRIEF DESCRIPTION OF THE DRAWINGS

Figure 1A is an elevational view of an obturator having features of the invention.
Figure 1B is a transverse cross sectional view of the obturator of Figure 1A taken along line 1B-1B.
Figure 1C is a longitudinal cross sectional view of the obturator of Figure 1A taken along lines 1C-1C.
Figure 1D is a longitudinal cross sectional view of an alternative obturator with a gel filled distal shaft portion similar to the view shown in Figure 1C.
Figure 1E is a longitudinal cross sectional view of another alternative obturator with a solid filled distal shaft portion similar to the view shown in Figure 1C.
Figures 2A-2C illustrate a marker delivery assembly which includes the obturator shown in Figure 1. Figure 2D is a longitudinal cross-sectional view of the distal portion of the assembly with the elements assembled.
Figure 3 is a perspective view of a substantially sealed distal end of an obturator having one or more petals.
Figure 4 is a perspective view of a substantially sealed distal end of an obturator having a duck billed valve.

### DETAILED DESCRIPTION OF EMBODIMENTS OF THE INVENTION

The present invention is directed to devices for marking an intracorporeal site within a patient's body, and particularly to an obturator having an MRI detectable mass in the distal shaft portion thereof for marking the intracorporeal site.

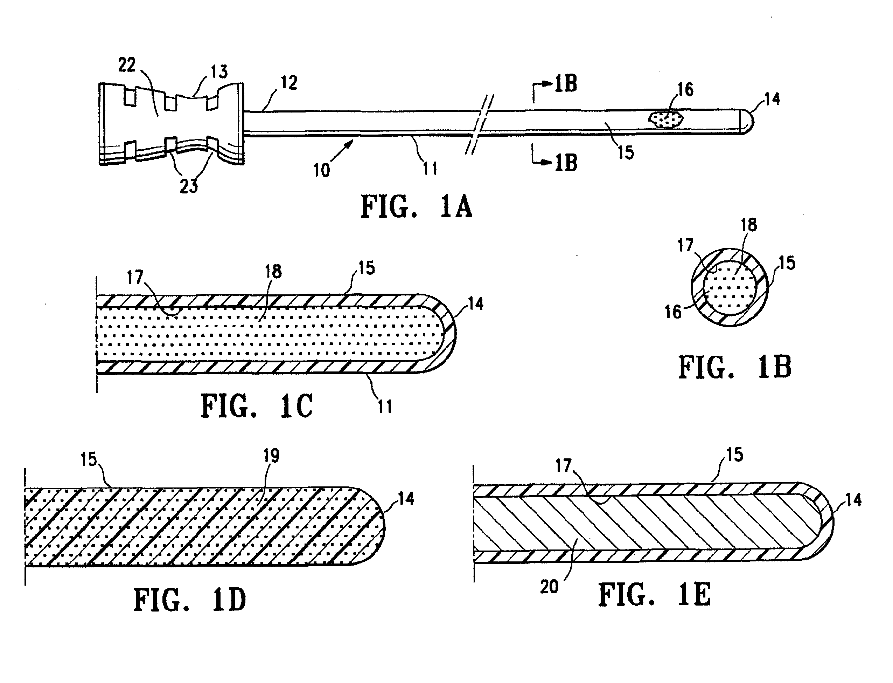

Figures 1A-C illustrate an embodiment of an obturator 10 having an elongated shaft 11, a proximal end 12, a handle 13 on the proximal end, a closed distal end 14 and a distal shaft portion 15 with an MRI detectable mass 16. The MRI detectable mass may be in a solid, semi-solid, e.g. a gel or a slurry, or liquid state. Non-flowable gels are presently preferred. As shown in Figures 1B and 1C, the distal shaft portion 15 has an inner lumen 17 that is filled with a gel 18 having an MRI detectable material incorporated therein. An alternative embodiment is shown in Figure 1D wherein the distal shaft portion 16 is solid 19 and has an MRI detectable material incorporated therein. Figure 1E depicts yet another alternative embodiment in which the distal shaft portion 15 has an inner lumen 17 that has a solid stylet 20, preferably formed of MRI detectable metallic material and preferably removable. The handle 13 has an outer surface 22 which facilitates gripping by the operator. In the embodiment shown, the outer surface is provided with a plurality of recesses 23 for gripping purposes. Alternatively, the surface of the handle 13 can be provided with raised portions or a roughened or other high friction surface to facilitate gripping by the operator. The obturator 10 is configured to fit within the lumen of a conventional trocar sheath (not shown) or the lumen or guide of a split sheath introducer such as shown in co-pending application Serial No. 60/946,079, filed on August 9, 2007.

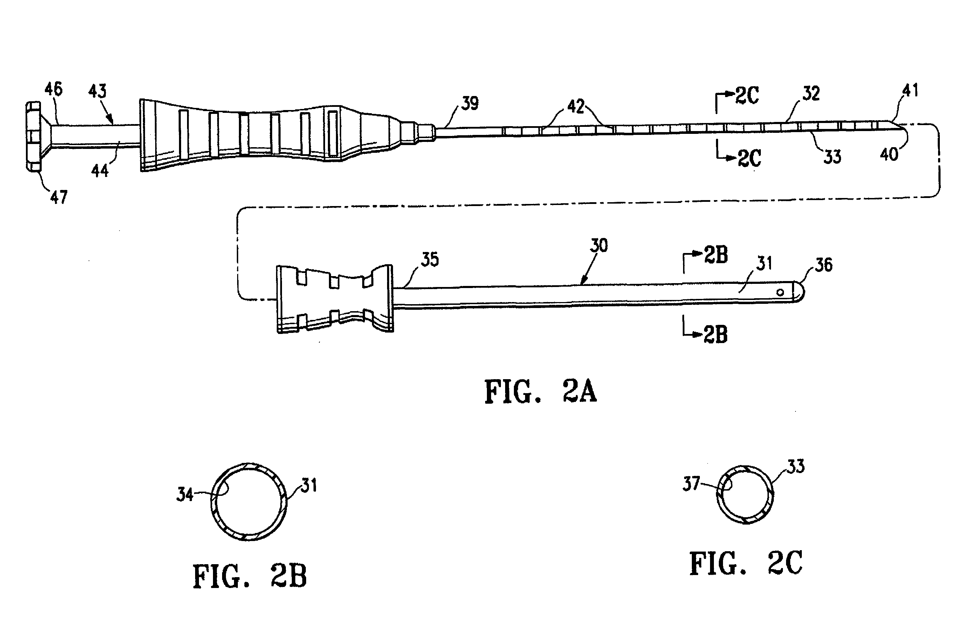

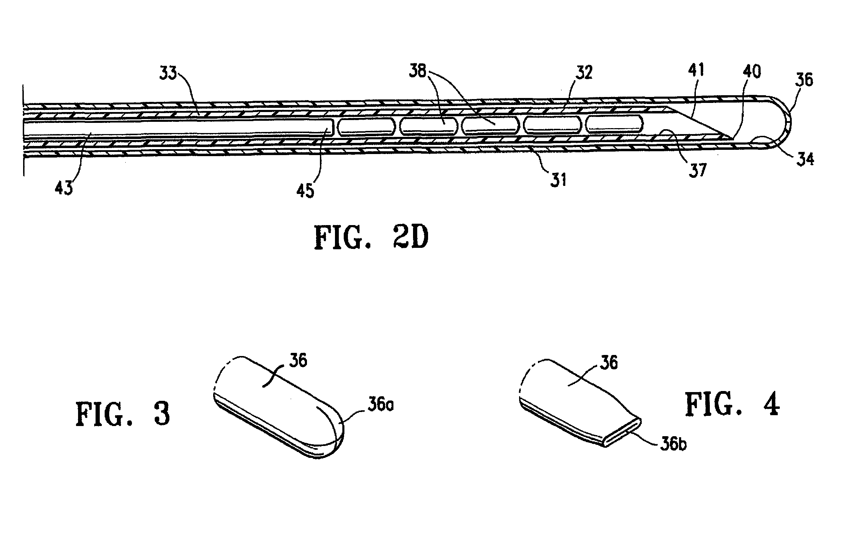

Figures 2A-2D shows an assembly having features of the invention including an obturator 30 with an MRI detectable distal shaft portion 31 and a marker delivery member 32 having a tubular shaft 33. The obturator 30 has an inner lumen 34, a proximal end 35 and a substantially closed distal end 36. The distal shaft portion 31 preferably has a removable MRI detectable element (not shown) such as a stylet shown in Figure1E. The distal end 36 of the obturator 30 is substantially closed to prevent or minimize the backflow of fluids, such as body fluids, through the inner lumen 34 of the obturator. Preferably, the substantially closed distal end 36 is a penetratable membrane. Alternatively, the substantially closed distal end 36 is formed of two or more petals 36a (Figure 3) or can be formed of a duck-billed valve 36b (Figure 4).

The marker delivery tubular shaft 33 is configured to be slidably disposed within the internal lumen 34 of the obturator 30. The tubular shaft 33 has a marker delivery lumen 37 configured to contain one or more tissue markers 38 for marking a biopsy site, a proximal end 39, and a distal tip 40 with an opening 41 for passage of one or more of the markers 38. The tissue markers 38 may be those described in U.S. Patent No. 6,996,433, U.S. Patent No. 6,993,375, U.S. Patent No. 6,862,470, U.S. Patent No. 6,725,083, U.S. Patent No. 6,662,041, U.S. Patent No. 6,567,689, U.S. Patent No. 6,427,081, 6,347,241, U.S. Patent No. 6,161,034, U.S. Patent Application No. 10/444,770, U.S. Patent Application No. 10/444,428, and U.S. Patent Application No. 10/001,043. The marker delivery tubular shaft 33 preferably also includes depth markings 42 which indicate the distance which the tubular shaft 33 has advanced within the obturator 30.

The distal tip 40 of the marker delivery shaft 33 is configured to penetrate the substantially closed distal end 36 of the obturator 30 so that tissue markers 38 can be delivered while the obturator 30 is in place within the patient's body. Preferably the distal tip 40 is needle shaped as shown. However, the distal tip 40 can alternatively be a blunt tip which is capable of penetrating the distal end 36 that is formed of a penetratable membrane which is weakened or a distal end with petals 36a or a valve 36b as shown in Figures 4 and 5.

The marker delivery member 32 also includes a plunger 43 having an elongated shaft 44 with a distal end 45 and a proximal end 46. The plunger 43 is configured to be slidably disposed within the marker delivery lumen 37 and is located proximal to the one or more tissue markers 38 within the marker delivery lumen 37. When the plunger 43 is extended distally within the marker delivery lumen 37 it moves one or more tissue markers 38 toward and eventually through the opening 41 in the distal tip 40 of the marker delivery member. The plunger 43 preferably has an enlarged head 47 to facilitate manual advancement. Alternatively, a fluid (not shown) may be used to advance the markers 38 through the opening 41 in the distal tip 40 of the marker delivery member.

Preferably, the obturator 30 is configured to fit within a cannula of a biopsy device, such as SenoRx's EnCor™ Magnetic Resonance Imaging (MRI) Breast Biopsy System. The cannula provides access to the desired location within a patient's body.

Further details of this assembly are shown in provisional application Serial No. 60/860,887, filed November 24, 2006, and provisional application Serial No. 60/872,020, filed November 30, 2006. Figures 6A-6C show the distal tip 40 of the marker delivery tube 33 partially penetrating the substantially closed distal end 36 of the obturator 30. The tissue markers 38 are contained within the marker delivery lumen 37. Figures 6A-6C show the distal tip 40 completely penetrating the substantially closed distal end 36 of the obturator 30 and the tissue markers 38 within the marker delivery lumen 37. In operation, the plunger 43 is deployed distally within the marker delivery lumen 37 so that the distal end 45 engages the markers 38 and advances the markers out the opening 41.

The marker delivery member 32 is preferably formed of a non-magnetic material. A polymeric material such as MAKROLON®, a polycarbonate from Bayer Material Sciences a division of Bayer AG, is suitable and will not interfere with a magnetic resonance imaging device (MRI). The device may also include a radiopaque material which allows for radiographic detection of the device.

Positive MRI contrast agents cause a reduction in the T1 relaxation time (increased signal intensity on T1 weighted images). They appear bright on MRI and are typically small molecular weight compounds containing as their active element Gadolinium, Manganese, or Iron. All of these elements have unpaired electron spins in their outer shells and long relaxivities. Some typical contrast agents include GdCl₃, Gd₂O₃ gadopentetate dimeglumine (Gd-DPTA), Gd-EDTA, gadoteridol, gadoterate meglumine, mangafodipir trisodium and gadodiamide. Negative MRI contrast agents appearing predominantly dark on MRI are small particulate aggregates often termed superparamagnetic iron oxide (SPIO). These MRI contrast agents produce predominantly spin spin relaxation effects (local field inhomogeneities), which results in shorter T1 and T2 relaxation times. SPIO's and ultrasmall superparamagnetic iron oxides (USPIO) usually consist of a crystalline iron oxide core containing thousands of iron atoms and a shell of polymer, dextran, polyethyleneglycol, and produce very high T2 relaxivities. USPIOs smaller than 300 nm cause a substantial T1 relaxation. T2 weighted effects are predominant.

In one embodiment, a lumen in the distal shaft portion of the obturator is filled with a gel having an MRI contrast material. One suitable gel is mineral oil in plastisol. Another is a paramagnetic contrast agent in a hydrogel. For example, the following aqueous solutions were prepared at the indicated molar concentrations:

| **MRI CONTRAST MATERIAL** | **MOLAR CONCENTRATION** |
|---|---|
| Gadiolinium Sulfate | 0.00002-0.0005 |
| Ferric Chloride | 0.00005-0.0005 |
| Ferrous Gluconate | 0.00005-0.001 |
| Manganese Chloride | 0.0001-0.0005 |

The aqueous solutions were mixed with polyvinyl alcohol cross-linked with boric acid to create a gel which is slightly flowable and less susceptible to bubble movement due to changes in position. Other gel forming materials include polyethylene glycol, gelatin, agar, polyHEMA, polyacrylamide, Pluronic F127. The formed gels need sufficient water to allow the water to react to the magnetic field during magnetic resonance imaging. To minimize loss of water during storage and the like, the gel may be protected with a material such as polypropylene which has essentially no water vapor transmission therethrough.

If an imageable stylet is used within the inner lumen of the obturator as shown in Figure 1E, it is preferably formed of material which is compatible with MRI and which is seen in MRI generated images without interfering with imaging of adjacent tissue. Suitable materials include non-magnetic metals, non-magnetic metal filled plastics, hollow tubes filled at least in part with an MRI visible substance such as described above.

While particular forms of the invention have been illustrated and described herein directed to detectable markers, it will be apparent that various modifications and improvements can be made to the invention. For example, the deployed bodies may be therapeutic or diagnostic agents in addition to or in lieu of being markers. Moreover, individual features may be shown or otherwise described in one embodiment and not in others, but those skilled in the art will recognize that individual features of one embodiment of the invention can be combined with any or all the features of another embodiment. Accordingly, it is not intended that the invention be limited to the specific embodiments illustrated. To the extent not otherwise disclosed herein, materials and structure may be of conventional design.

## Claims

1. A combination of an obturator (30) for marking an intracorporeal site and a marker delivery member (32) for insertion in the obturator,
the obturator comprising:
an elongated shaft having a proximal end (35), and a closed distal end (36), and an MRI detectable distal shaft portion (31) which does not interfere with magnetic resonance imaging of tissue proximate thereto, wherein the distal shaft portion comprises an inner lumen (34) containing a removable stylet (20) formed of or containing MRI detectable material,
the marker delivery member comprising:
an elongated shaft (33),
an inner lumen (37) extending within the shaft configured to contain one or more tissue markers (38) within the inner lumen, and
a plunger (43) within the lumen which is configured to be slidably disposed within the inner lumen and which is located proximal to the one or more markers,
a distal tip (40) of the elongated shaft being configured to penetrate said closed distal end of the obturator so that the tissue markers can be delivered.

2. The combination of claim 1 wherein the distal shaft portion has MRI contrast agent therein.

3. The combination of claim 1 wherein the distal shaft portion has an effective amount of MRI detectable agent so as to provide a clear, T1-weighted image within an outline of the distal shaft portion upon magnetic resonance imaging thereof.

4. The combination of claim 2 wherein the MRI detectable agent is selected from the group consisting of MRI detectable agents containing gadolinium, manganese, or iron.

5. The combination of claim 2 wherein the contrast agent is selected from the group consisting of GdCl, Gd₂O₃ gadopentetate dimeglumine (Gd-DPTA), Gd-EDTA, gadoteridol, gadoterate meglumine, mangafodipir trisodium and gadodiamide

6. The combination of claim 3 wherein the distal shaft portion has an inner lumen and an MRI detectable mass within the inner lumen containing MRI detectable agent.

7. The combination of claim 1 wherein the stylet is formed of polymeric material.

8. The combination of claim 1 wherein the closed distal end is a penetratable membrane.

9. The combination of claim 1 wherein the closed distal end of the obturator is formed of one or more petals (36a).

10. The combination of claim 1 wherein the closed distal end of the obturator is a valve (36b).

11. The combination of claim 1 wherein the proximal end of the obturator has a hub and the proximal end of the marker delivery member has a hub which is configured to fit within the hub of the obturator when the marker delivery shaft is inserted into the elongated shaft of the obturator.

12. The combination of claim 1 wherein the marker delivery lumen contains a fluid proximal of the at least one tissue marker to facilitate discharge of the markers.

13. The combination of claim 1 wherein the obturator shaft is formed of a nonmagnetic material.

14. The combination of claim 1 wherein the polymeric material is a polycarbonate.

15. The combination of claim 1 wherein the obturator shaft is configured to be used within a cannula of a biopsy device.

## Patentansprüche

1. Kombination aus einem Obturator (30) zur Markierung einer Stelle innerhalb eines Körpers und einem Marker-Ausbringelement (32) zum Einführen in den Obturator,
wobei der Obturator Folgendes umfasst:
einen länglichen Schaft mit einem proximalen Ende (35) und einem verschlossenen distalen Ende (36) und einen MRT-erfassbaren distalen Schaftabschnitt (31), der die Magnetresonanztomografie von in der Nähe liegendem Gewebe nicht beeinflusst, wobei der distale Schaftabschnitt ein inneres Lumen (34) umfasst, das einen entfernbaren Mandrin (20) enthält, der aus MRT-erfassbarem Material gebildet ist oder dieses enthält,
wobei das Marker-Ausbringelement Folgendes umfasst:
einen länglichen Schaft (33),
ein inneres Lumen (37), das sich im Schaft erstreckt und dazu konfiguriert ist, einen oder mehrere Gewebe-Marker (38) im inneren Lumen zu enthalten, und
einen Stößel (43) im Lumen, der dazu konfiguriert ist, gleitend im inneren Lumen angeordnet zu sein, und der proximal zu dem einen oder den mehreren Markern angeordnet ist,
wobei eine distale Spitze (40) des länglichen Schafts dazu konfiguriert ist, das verschlossene distale Ende des Obturators zu durchdringen, so dass die Gewebe-Marker ausgebracht werden können.

2. Kombination nach Anspruch 1, wobei der distale Schaftabschnitt MRT-Kontrastmittel enthält.

3. Kombination nach Anspruch 1, wobei der distale Schaftabschnitt eine wirksame Menge an MRT-nachweisbarem Mittel hat, um ein klares T1-gewichtetes Bild in einem Umriss des distalen Schaftabschnitts bei der Magnetresonanztomografie davon bereitzustellen.

4. Kombination nach Anspruch 2, wobei das MRT-nachweisbare Mittel aus der aus MRT-nachweisbaren Mitteln, die Gadolinium, Mangan oder Eisen enthalten, bestehenden Gruppe ausgewählt ist.

5. Kombination nach Anspruch 2, wobei das Kontrastmittel aus der aus GdCl, Gd₂O₃, Gadopentetat-Dimeglumin (Gd-DPTA), Gd-EDTA, Gadoteridol, Gadoterat-Meglumin, Mangafodipir Trisodium und Gadodiamid bestehenden Gruppe ausgewählt ist.

6. Kombination nach Anspruch 3, wobei der distale Schaftabschnitt ein inneres Lumen hat sowie eine MRT-nachweisbare Masse im inneren Lumen, die MRT-nachweisbares Mittel enthält.

7. Kombination nach Anspruch 1, wobei der Mandrin aus polymerem Material gebildet ist.

8. Kombination nach Anspruch 1, wobei das verschlossene distale Ende eine durchdringbare Membran ist.

9. Kombination nach Anspruch 1, wobei das verschlossene distale Ende des Obturators aus einem oder mehreren Petalen (36a) gebildet ist.

10. Kombination nach Anspruch 1, wobei das verschlossene distale Ende des Obturators ein Ventil (36b) ist.

11. Kombination nach Anspruch 1, wobei das proximale Ende des Obturators eine Nabe hat und das proximale Ende des Marker-Ausbringelements eine Nabe hat, die dazu konfiguriert ist, in die Nabe des Obturators zu passen, wenn der Marker-Ausbringschaft in den länglichen Schaft des Obturators eingeführt wird.

12. Kombination nach Anspruch 1, wobei das Marker-Ausbringlumen proximal des mindestens einen Gewebe-Markers ein Fluid enthält, um das Austragen der Marker zu erleichtern.

13. Kombination nach Anspruch 1, wobei der Obturatorschaft aus einem nicht magnetischen Material gebildet ist.

14. Kombination nach Anspruch 1, wobei das polymere Material ein Polycarbonat ist.

15. Kombination nach Anspruch 1, wobei der Obturatorschaft dazu konfiguriert ist, in einer Kanüle einer Biopsievorrichtung verwendet zu werden.

## Revendications

1. Combinaison d'un obturateur (30) pour marquer un site intra-corporel et d'un élément de distribution de marqueur (32) à insérer dans l'obturateur,
l'obturateur comprenant:
un arbre allongé présentant une extrémité proximale (35), et une extrémité distale fermée (36), et une partie d'arbre distale détectable par IRM (31) qui n'interfère pas avec l'imagerie par résonance magnétique du tissu à proximité de celle-ci, dans laquelle la partie d'arbre distale comprend une lumière intérieure (34) contenant un stylet amovible (2) constitué d'un matériau détectable par IRM ou contenant un matériau détectable par IRM;
l'élément de distribution de marqueur comprenant:
un arbre allongé (33),
une lumière intérieure (37) qui s'étend à l'intérieur de l'arbre et est configurée de manière à contenir un ou plusieurs marqueur(s) de tissu (38) à l'intérieur de la lumière intérieure, et
un plongeur (43) à l'intérieur de la lumière qui est configuré de manière à être disposé de façon coulissante à l'intérieur de la lumière intérieure et qui est situé à proximité dudit/desdits un ou plusieurs marqueur(s),
une pointe distale (40) de l'arbre allongé étant configurée de manière à pénétrer dans ladite extrémité distale fermée de l'obturateur de telle sorte que les marqueurs de tissu puissent être distribués.

2. Combinaison selon la revendication 1, dans laquelle la partie d'arbre distale comprend un agent de contraste pour IRM dans celle-ci.

3. Combinaison selon la revendication 1, dans laquelle la partie d'arbre distale contient une quantité effective d'un agent détectable par IRM de manière à fournir une image nette pondérée en T1 à l'intérieur d'un contour de la partie d'arbre distale lors d'une imagerie par résonance magnétique de celle-ci.

4. Combinaison selon la revendication 2, dans laquelle l'agent détectable par IRM est sélectionné dans le groupe comprenant des agents détectables par IRM contenant du gadolinium, du manganèse ou du fer.

5. Combinaison selon la revendication 2, dans laquelle l'agent de contraste est sélectionné dans le groupe comprenant le GdCl, le Gd₂O₃, le gadopentétate de diméglumine (Gd-DPTA), le Gd-EDTA, le gadotéridol, le gadotérate de méglumine, le mangafodipir trisodium et le gadodiamide.

6. Combinaison selon la revendication 3, dans laquelle la partie d'arbre distale comprend une lumière intérieure et une masse détectable par IRM à l'intérieur de la lumière intérieure contenant un agent détectable par IRM.

7. Combinaison selon la revendication 1, dans laquelle le stylet est constitué d'un matériau polymère.

8. Combinaison selon la revendication 1, dans laquelle l'extrémité distale fermée est une membrane pénétrable.

9. Combinaison selon la revendication 1, dans laquelle l'extrémité distale fermée de l'obturateur est constituée d'un ou de plusieurs pétale(s) (36a).

10. Combinaison selon la revendication 1, dans laquelle l'extrémité distale fermée de l'obturateur est une valvule (36b).

11. Combinaison selon la revendication 1, dans laquelle l'extrémité proximale de l'obturateur comporte un moyeu, et l'extrémité proximale de l'élément de distribution de marqueur comprend un moyeu qui est configuré de manière à s'agencer à l'intérieur du moyeu de l'obturateur lorsque l'arbre de distribution de marqueur est inséré dans l'arbre allongé de l'obturateur.

12. Combinaison selon la revendication 1, dans laquelle la lumière de distribution de marqueur contient un fluide à proximité dudit au moins un marqueur de tissu afin de faciliter la décharge des marqueurs.

13. Combinaison selon la revendication 1, dans laquelle l'arbre d'obturateur est constitué d'un matériau non magnétique.

14. Combinaison selon la revendication 1, dans laquelle le matériau polymère est un polycarbonate.

15. Combinaison selon la revendication 1, dans laquelle l'arbre d'obturateur est configuré de manière à être utilisé à l'intérieur d'une canule d'un dispositif de biopsie.
